## Europäisches Patentamt
## European Patent Office
## Office européen des brevets

(11) Publication number: **0 083 674**
**B1**

(12) **EUROPEAN PATENT SPECIFICATION**

(45) Date of publication of patent specification: **20.11.86**

(51) Int. Cl.⁴: **A 61 N 1/04**

(21) Application number: **82100142.7**

(22) Date of filing: **11.01.82**

(54) Temporary lead with insertion tool.

(43) Date of publication of application:
**20.07.83 Bulletin 83/29**

(45) Publication of the grant of the patent:
**20.11.86 Bulletin 86/47**

(84) Designated Contracting States:
**DE FR NL SE**

(56) References cited:
**EP-A-0 049 780**
**DE-A-2 846 136**
**FR-A-2 013 858**
**FR-A-2 431 295**
**US-A-3 125 095**
**US-A-3 216 424**
**US-A-3 978 865**

(73) Proprietor: **MEDTRONIC, INC.**
**3055 Old Highway Eight**
**Minneapolis Minnesota 55440 (US)**

(72) Inventor: **Peters, Peter**
**Albert Schweitzerstraat 46**
**6441 HG Brussum (NL)**

(74) Representative: **Schwan, Gerhard, Dipl.-Ing.**
**Elfenstrasse 32**
**D-8000 München 83 (DE)**

Courier Press, Leamington Spa, England.

## Description

The present invention relates generally to a surgical electrode lead and more specifically relates to a lead for temporary application.

The use of temporary leads for pacing and monitoring purposes is quite common. Specially designed leads are used for such temporary applications which are much lighter and less durable than permanent leads since extended flex life is not required. It is still critical, however, that electrodes be properly affixed to tissue to permit the required transfer of electrical energy. This electrical contact must be established in a manner which permits convenient and safe removal of the lead with minimal permanent scarring and other effects. Furthermore, for epicardial applications, most permanent leads are more costly than is felt justified by temporary use.

Construction of temporary leads for curing cardiac arrest are known from US—A—3 485 247 and US—A—3 516 412. The former reference uses a hook-shaped tip for affixing the lead whereas the latter uses resiliency of shape. Neither of these techniques is suitable for most applications, however, as both leads are intended to be percutaneously inserted and actually puncture the myocardium. Because of the permanent effects of this technique, it is not useful under routine circumstances.

The primary method of affixing temporary epicardial leads is with sutures. Typically, this technique provides the greatest reliability with minimal permanent damage. Sutures were used in the earliest pacing applications for affixing all leads. US—A—3 244 174 teaches a lead whose electrodes are affixed using a suture pad.

US—A—3 474 791 teaches a lead having insulation removed at points which permit electrical contact. The lead may have a curved surgical needle attached directly to the distal end of the conductor for sticking the lead directly into the myocardium. Additional sutures are used to further attach the lead to the epicardium.

These earlier suturing techniques for affixing the electrode to the myocardium lend themselves primarily to permanent implantation, since removal of the lead is difficult.

DE—A—2 846 136 discloses a lead for temporary or permanent application having an insulated conductor. A connector and an enlarged electrode head are attached to the proximal end and to the distal end, respectively, of the conductor. The proximal end of a surgical thread is fixed to the electrode head, and a curved needle is attached to the distal end of the surgical thread.

Furthermore it is known from FR—A—2 431 295 to provide a stimulating lead with undulations. The undulated part of the lead is straightened by a stylet during insertion of the lead into tissue. The stylet is later removed thereby allowing anchoring of the lead in the tissue.

The present invention provides for a lead for establishing electrical contact between body tissue and a medical device comprising:

a length of conductor having a proximal end and a distal end;

a sheath attached to the surface of said length of conductor;

a connector fixedly attached to said proximal end of said length of conductor whereby said lead may be electrically coupled to said medical device;

an electrode fixedly attached to said distal end of said length of conductor;

a length of surgical thread having a proximal end and a distal end wherein said proximal end of said length of surgical thread is fixedly attached to said electrode; and

a needle fixedly attached to said distal end of said length of surgical thread;

which lead is characterized by

frictional resisting means comprising portion of said length of surgical thread having the general shape of a helix which is coiled in the vicinity of the proximal end of surgical thread.

The lead according to the present invention particularly is suited for temporary epicardial stimulation or monitoring. The lead is less expensive than permanent leads, being intended for short-term use only. A single suture affixes the electrode to the epicardium. The length of surgical thread and the needle, preferably a curved needle, are permanently affixed to the electrode at the distal end of the lead. The helix preferably is molded into the surgical thread a short distance from the electrode.

The curved needle enters and exits the epicardium and the surgical thread is manually pulled until the electrode actually enters to tissue. The helix within the surgical thread is spaced a distance from the electrode ensuring that it is located within the myocardium. The pulling tension on the surgical thread elongates the helix. With tension on the surgical thread, the excess surgical thread is cut at the point of exiting the epicardium. Cutting the surgical thread releases the tension on the helix tending to allow it to return to its original shape. This tendency of the helix holds the electrode in place. Retreat of the severed end of the surgical thread permits immediate healing at the location at which the needle exited the tissue.

After the temporary lead is no longer needed, the lead is removed by pulling on the proximal end. The tension thus created tends to elongate the helix thereby easing removal.

One embodiment of the invention is described in detail below with reference to the drawings, in which

Fig. 1 is a view of the entire temporary lead,

Fig. 2 is a side sectional view of the electrode with surgical thread and helix attached,

Fig. 3 shows the curved needle as inserted into the myocardium at proper depth,

Fig. 4 shows elongation of the helix as the electrode is pulled into position,

Fig. 5 shows cutting off excess surgical thread,

Fig. 6 is a sectional view of the myocardial tissue with tension applied to surgical thread, and

Fig. 7 is a sectional view of the myocardial tissue with tension removed.

The present invention is disclosed as embodied in a temporary pacing lead and associated insertion tool. Experiments have indicated that manual insertion of the curved needle is most satisfactory when using the lead for ventricular pacing. However, those of ordinary skill in the art will be readily able to utilize the present invention in other applications and with different dimensions.

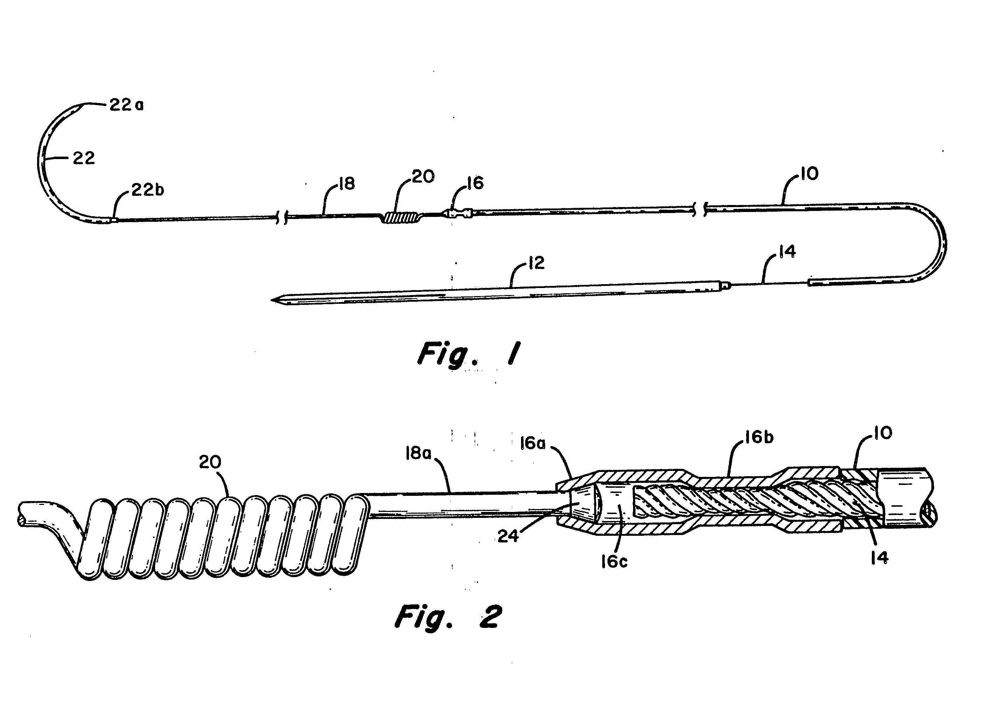

Fig. 1 shows the temporary pacing lead. The proximal end contains a metallic, electrically conductive needle 12 which is used as a connector pin to couple the lead to a pulse generator. Needle 12 is attached to the flexible conductor 14 which runs the length of the temporary pacing lead and is attached to electrode 16. Sheath 10 is of a material substantially inert to bodily fluids such as polyethylene. Sheath 10 also electrically insulates conductor 14. A length of surgical thread 18 is permanent attached to electrode 16. Surgical thread 18 has an outside diameter of about 0.35 mm and is a commonly available type such as polypropylene. Helix 20 is molded into the surgical thread by applying hor air to the coiled surgical thread. Curved needle 22 is a standard surgical —3/8 circle needle of 12.0 mm radius. The surgical thread is attached to the curved needle by crimp 22b. Needle point 22a is hand honed.

Fig. 2 is a sectional view of electrode 16. The total length of electrode 16 is about 3 mm. The outside diameter of Sheath 10 is approximately 0.7 mm. Sheath 10 extends to the proximal end of electrode 16. Conductor 14 extends distal relative to Sheath 10 and into electrode 16 as shown. Electrode 16 is crimped to securely connect to conductor 14 at indentation 16b. The proximal end of surgical thread 18 is enlarged to produce fastener 24. This can be accomplished by heating the proximal end of surgical thread 18 and applying a force in the distal direction, thus flattening the end.

Fastener 24 is inserted into chamber 16c which lies within electrode 16 between the distal end of conductor 14 and the distal end of electrode 16. The distal end of electrode 16 is swaged producing the conical frustum 16a, which securely holds fastener 24. Conical frustum 16a also helps ease insertion of electrode 16 into epicardial tissue.

Between conical frustum 16a and helix 20 is a length of surgical thread 18a being about 1.0 mm. Helix 20 is about 9—11 turns having an outside diameter of approximately 1.0 mm.

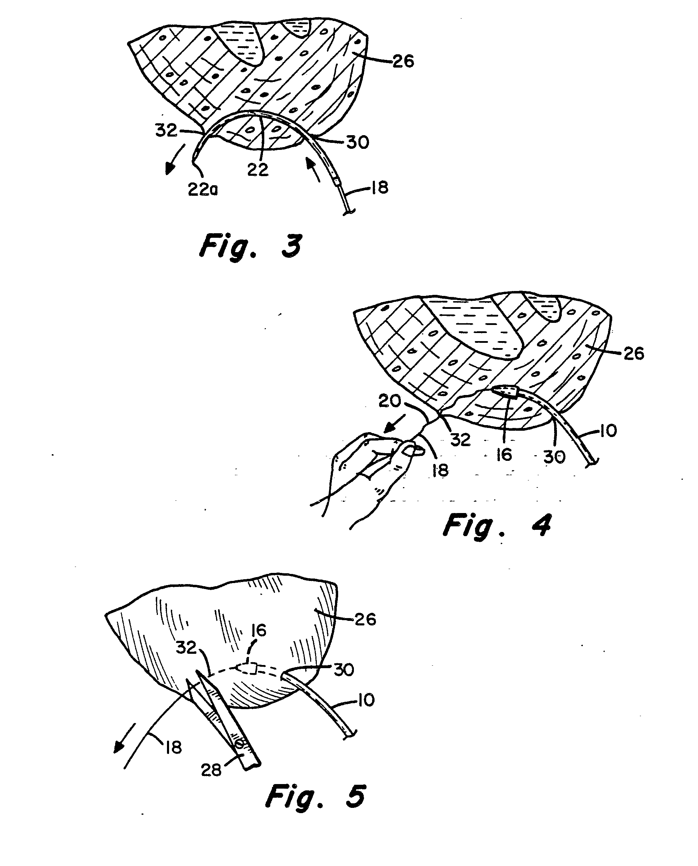

Fig. 3 shows proper insertion of curved needles 22. This may be accomplished manually or through the use of the insertion tool described below. Myocardium 26 is entered at first location 30 and exited at second location 32 by curved needle 22 being moved in the direction shown.

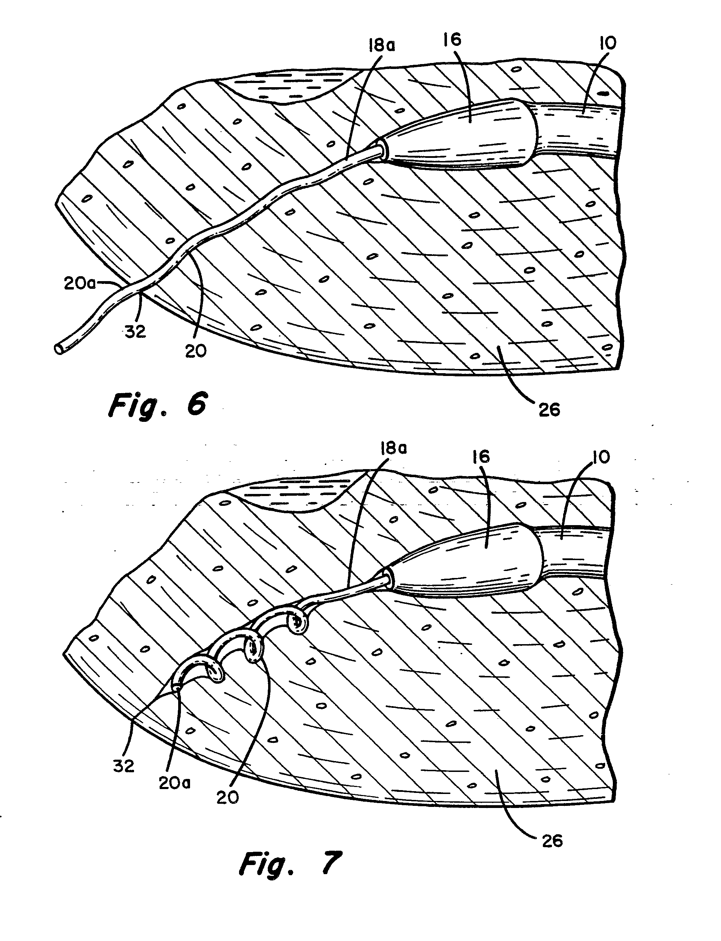

Fig. 4 shows the manner in which electrode 16 is properly positioned. Surgical thread 18 is manually pulled from second location 32 until electrode 16 to which it is attached is pulled into the orifice at location 30. Notice that electrode 16 is located completely within myocardial tissue. Because of the stress on helix 20 caused by pulling surgical thread 18 in the direction shown and the friction between electrode 16 and the myocardial tissue, helix 20 becomes temporarily elongated as shown. Fig. 6 shows an elarged sectional view of myocardial tissue 26 with electrode 16 properly positioned. Notice that a portion of elongated helix 20 extends from the orifice at second location 32.

Fig. 5 shows removal of the excess surgical thread. While tension is continuously supplied to surgical thread 18 maintaining helix 20 in its elongated state, surgical thread 18 is severed by cutting instrument 28 near second location 32. Fig. 7 is an enlarged sectional view of myocardial tissue 26 following severing of the excess surgical thread. Notice that whereas helix 20 does not return to its initial shape, its longitudinal distance is somewhat reduced causing the tension with the surrounding myocardial tissue which continues to hold electrode 16 in place. Also notice that helix 20 was severed at point 20a. Severing the surgical thread at helix 20 ensures that point 20a will retreat from second location 32 into the myocardial tissue which permits immediate healing of second location 32.

To remove the temporary pacing lead, tension is applied by pulling the proximal end of the temporary pacing lead. The tension tends to elongate helix 20 easing removal. Notice that healing need occur only at a single orifice (i.e., at first location 30) upon removal as the orifice at second location 32 begins healing immediately.

**Claims**

1. A lead for establishing electrical contact between body tissue and a medical device comprising:
   a length of conductor (14) having a proximal end and a distal end;
   a sheath (10) attached to the surface of said length of conductor;
   a connector (12) fixedly attached to said proximal end of said length of conductor whereby said lead may be electrically coupled to said medical device;
   an electrode (16) to said distal end of said length of conductor;
   a length of surgical thread (18) having a proximal end and a distal end wherein said proximal end of said length of surgical thread is fixedly attached to said electrode (16); and
   a needle (22) fixedly attached to said distal end of said length of surgical thread (18), characterized by frictionally resisting means comprising portion of said length of surgical thread (18) having the general shape of a helix (20) which is coiled in the vicinity of the proximal end of surgical thread (18).

2. A lead according to claim 1 wherein the helix is molded into the surgical thread (18).

3. A lead according to claim 1 or 2 wherein the proximal end of the surgical thread (18) is enlarged to form a fastener (24) which is inserted into a chamber (16c) within said electrode (16).

4. A lead according to claim 3 wherein the distal end of the electrode (16) is swaged to form a conical frustum (16a) holding said fastener (24).

5. A lead according to any one of claims 1 to 4 wherein said helix (20) has about 9 to 11 turns.

6. A lead according to any one of claims 1 to 5, wherein said helix (20) has an outside diameter of approximately 1 mm.

## Patentansprüche

1. Leitung zum Herstellen von elektrischem Kontakt zwischen Körpergewebe und einem medizinischen Gerät mit:

einem Stück eines Leiters (14) mit einem proximalen Ende und einem distalen Ende;

einer an der Oberfläche des Leiterstückes angebrachten Ummantelung (10);

einem an dem proximalen Ende des Leiterstückes fest angebrachten Verbindungsglied (12), wodurch die Leitung mit dem medizinischen Gerät elektrisch gekoppelt werden kann;

einer an dem distalen Ende des Leiterstückes fest angebrachten Elektrode (16);

einem Stück chirurgischem Faden (18) mit einem proximalen Ende und einem distalen Ende, wobei das proximale Ende des chirurgischen Fadenstückes an der Elektrode (16) fest angebracht ist; und

einer an dem distalen Ende des Stückes des chirurgischen Fadens (18) fest angebrachten Nadel (22), gekennzeichnet durch

Reibungswiderhaltmittel mit einem Teil des Stückes des chirurgischen Fadens (18), der im wesentlichen die Form einer Wendel (20) hat, die in der Nähe des proximalen Endes des chirurgischen Fadens (18) gewickelt ist.

2. Leitung nach Anspruch 1, wobei die Wendel (20) in den chirurgischen Faden (18) eingeformt ist.

3. Leitung nach Anspruch 1 oder 2, wobei das proximale Ende des chirurgischen Fadens (18) zur Bildung eines Befestigungsgliedes (24) vergrößert ist, das in eine Kammer (16c) in der Elektrode (16) eingesetzt ist.

4. Leitung nach Anspruch 3, wobei das distale Ende der Elektrode (16) eingeschnürt ist, um einen das Befestigungsglied (24) haltenden Kegelstumpf (16a) zu bilden.

5. Leitung nach einem der Ansprüche 1 bis 4, wobei die Wendel (20) etwa 9 bis 11 Windungen hat.

6. Leitung nach einem der Ansprüche 1 bis 5, wobei die Wendel (20) einen Außendurchmesser von näherungsweise 1 mm hat.

## Revendications

1. Sonde permettant d'établir un contact électrique entre un tissu corporel et un appareil médical, comprenant un tronçon de conducteur (14) présentant une extrémité proximale et une extrémité distale, une gaine (10) fixée à la surface dudit tronçon de conducteur, un organe de connexion (12) fixé à demeure sur ladite extrémité proximale dudit tronçon de conducteur, de sorte que ladite sonde peut être accouplée électriquement audit appareil médical, une électrode (16) fixée à demeure sur ladite extrémité distale dudit tronçon de conducteur, un tronçon de fil chirurgical (18) présentant une extrémité proximale et une extrémité distale, ladite extrémité proximale dudit tronçon de fil chirurgical étant fixée à demeure sur ladite électrode (16), et une aiguille (22) fixée à demeure sur ladite extrémité distale dudit tronçon de fil chirurgical (18), caractérisée par des moyens à résistance par friction comprenant une partie dudit tronçon de fil chirurgical (18) ayant la forme générale d'un hélice (20) qui est enroulée au voisinage de l'extrémité proximale du fil chirurgical (18).

2. Sonde selon la revendication 1, dans laquelle l'hélice (20) est moulée dans le fil chirurgical (18).

3. Sonde selon la revendication 1 ou 2, dans laquelle l'extrémité proximale du fil chirurgical (18) est élargie de façon à former une attache (24) qui est insérée dans une chambre (16c) située à l'intérieur de ladite électrode (16).

4. Sonde selon la revendication 3, dans laquelle l'extrémité distale de l'électrode (16) est rétreinte de façon à former un tronc de cone (16a) maintenant ladite attache (24).

5. Sonde selon l'une quelconque des revendications 1 à 4, dans laquelle ladite hélice (20) présente environ 9 à 11 tours.

6. Sonde selon l'une quelconque des revendications 1 à 5, selon laquelle ladite hélice (20) présente un diamètre extérieur d'approximativement 1 mm.

Fig. 1

Fig. 2

0 083 674

**Fig. 3**

**Fig. 4**

**Fig. 5**

Fig. 6

Fig. 7